(19) **Europäisches Patentamt / European Patent Office / Office européen des brevets**

(11) **EP 4 620 946 A1**

(12) **EUROPEAN PATENT APPLICATION**
published in accordance with Art. 153(4) EPC

(43) Date of publication:
24.09.2025 Bulletin 2025/39

(21) Application number: 23890879.2

(22) Date of filing: 17.11.2023

(51) International Patent Classification (IPC):
*C07C 253/20* (2006.01)    *C07C 255/10* (2006.01)
*B01J 21/06* (2006.01)    *B01J 23/08* (2006.01)
*B01J 23/14* (2006.01)    *B01J 23/28* (2006.01)
*B01J 23/30* (2006.01)    *B01J 23/36* (2006.01)
*B01J 23/46* (2006.01)

(52) Cooperative Patent Classification (CPC):
(C-Sets available)
**C07C 253/20; B01J 21/06; B01J 23/08;
B01J 23/14; B01J 23/28; B01J 23/30; B01J 23/36;
B01J 23/46**    (Cont.)

(86) International application number:
**PCT/CN2023/132195**

(87) International publication number:
**WO 2024/104450 (23.05.2024 Gazette 2024/21)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 18.11.2022 CN 202211444469

(71) Applicants:
• **Zhejiang Research Institute of Chemical Industry
Co., Ltd.**
**Hangzhou, Zhejiang 310023 (CN)**
• **Sinochem Lantian Co., Ltd.**
**Hangzhou, Zhejiang 310051 (CN)**

(72) Inventors:
• **MAO, Chongzhi**
**Hangzhou, Zhejiang 310023 (CN)**
• **NI, Hang**
**Hangzhou, Zhejiang 310023 (CN)**
• **JIANG, Qiang**
**Hangzhou, Zhejiang 310023 (CN)**
• **YANG, Wangsong**
**Hangzhou, Zhejiang 310023 (CN)**
• **CHEN, Wei**
**Hangzhou, Zhejiang 310023 (CN)**
• **LI, Wei**
**Hangzhou, Zhejiang 310023 (CN)**

(74) Representative: **Viering, Jentschura & Partner
mbB
Patent- und Rechtsanwälte
Am Brauhaus 8
01099 Dresden (DE)**

(54) **METHOD FOR PREPARING HEPTAFLUOROISOBUTYRONITRILE BY GAS-PHASE CATALYSIS**

(57)    The present application provides a method of preparing heptafluoroisobutyronitrile by a gas-phase catalysis. The method includes removing one water molecule from heptafluoroisobutyramide gas to obtain heptafluoroisobutyronitrile by a catalyst. The catalyst includes an oxide of a siderophile element, and the siderophile element includes a moderately siderophile element or a highly siderophile element. The moderately siderophile element is selected from the group consisting of tungsten, molybdenum, tin, gallium, and any combination thereof, and the highly siderophile element is selected from the group consisting of osmium, iridium, ruthenium, rhenium, titanium, and any combination thereof. In the present application, the catalyst is utilized for catalytic dehydration, a cost is low, a water waste, a gas waste and a solid waste are less, a gas-phase continuous reaction can be achieved, and the method is suitable for industrial production. Furthermore, the catalyst has a high catalytic activity and a long catalytic life.

EP 4 620 946 A1

FIG. 1

(52) Cooperative Patent Classification (CPC): (Cont.)

C-Sets
**C07C 253/20, C07C 255/10**

**Description**

TECHNICAL FIELD

The present application relates to a preparation of heptafluoroisobutyronitrile, and in particular, to a method for preparing heptafluoroisobutyronitrile by a gas-phase catalysis.

BACKGROUND

Heptafluoroisobutyronitrile (C4F7N) have a low toxicity and an excellent chemical stability, a global warming potential (GWP) thereof is 2400 which is 1/10 of that of sulfur hexafluoride, and an ozone depletion potential thereof is 0. Compared with the sulfur hexafluoride, a life of C4F7N is greatly shorten in an atmosphere, and it is possible of replacing sulfur hexafluoride by C4F7N. At present, heptafluoroisobutyronitrile is obtained by dehydrating heptafluoroisobutyramide. A method of dehydrating the heptafluoroisobutyramide is mainly a dehydration method by a dehydrating agent.

Patent No. US15997808A discloses a method including following steps: selecting methylheptafluoroisobutyrate and an ammonia gas as a raw material to prepare heptafluoroisobutyramide, and performing a dehydration reaction using pyridine and trifluoroacetic anhydride as dehydrating agents to obtain heptafluoroisobutyronitrile; and in the dehydration reaction, a yield of heptafluoroisobutyronitrile is 74.9%. Patent No. CN108395382A discloses a method for preparing heptafluoroisobutyronitrile using trifluoroacetic anhydride as a dehydrating agent, and a yield of heptafluoroisobutyronitrile is 76%. Patent No. CN110642750A discloses a method for preparing heptafluoroisobutyronitrile by dehydrating perfluoroalkyl amide using a combination of fluorinated carboxylic anhydride (such as trifluoroacetic anhydride, pentafluoropropionic anhydride, heptafluorobutyric anhydride, heptafluoroisobutyric or anhydride) and N,N-Dimethylformamide as a dehydrating agent. Patent No. CN107935884A discloses that during a process of dehydrating heptafluoroisobutyramide, a dehydrating agent is mainly selected from the group consisting of trifluoroacetic anhydride, acetic anhydride, thionyl chloride, phosphorus pentoxide, phosphorus oxychloride and any combination thereof, and an organic solvent is selected from the group consisting of pyridine, polyphosphoric acid, carbon tetrachloride, N, N-dimethylformamide, N, N-diethylformamide, 1,4-dioxane, dimethyl sulfoxide and any combination thereof.

A process of dehydrating heptafluoroisobutyramide by the dehydrating agent is mature, but the process has following defects: (1) belonging to an intermittent process, it is difficult to achieve a large-scale continuous production; (2) using a large amount of a dehydrating agent, it is prone to generating a large amount of a liquid waste or a solid waste, and a consumed dehydrating agent is difficult to be recycled and reused; and (3) using a large amount of solvents, but due to an introduction of a dehydrating agent and a by-product thereof, the solvents are difficult to be recycled and reused, resulting in a large amount of a liquid waste and a serious environmental pollution.

Therefore, research for dehydrating heptafluoroisobutyramide is gradually emerging.

Patent No. CN114105820A discloses a method of dehydrating heptafluoroisobutyramide using a molecular sieve catalyst to obtain heptafluoroisobutyronitrile. The molecular sieve catalyst is selected from the group consisting of HY molecular sieve, NaY molecular sieve, β molecular sieve, ZSM-5 molecular sieve, 3A molecular sieve, 4A molecular sieve, 5A molecular sieve, and any combination thereof.

Patent No. CN114057605A discloses a method for preparing nitrile by a gas-phase catalytic dehydration, in which a dehydration catalyst used in the method is a supported catalyst. A carrier of the dehydration catalyst is selected from the group consisting of silica, molecular sieve, activated carbon, diatomaceous earth, montmorillonite, magnesium oxide, chromium oxide, iron oxide, nickel oxide, zinc oxide, and any combination thereof. An active component of the dehydration catalyst is selected from the group consisting of cobalt chloride, chromium chloride, zinc chloride, nickel chloride, iron chloride, copper chloride, and any combination thereof.

Patent No. CN109320436A discloses a method for preparing perfluorocyanide, in which a catalyst in a dehydrating reaction is selected from the group consist of aluminum oxide, copper oxide, cobalt oxide, niobium oxide, and any combination thereof.

Although the above methods may reduce an amount of three types of waste (liquid, solid, and gas) by more than 90%, and a cost by a range of 30% to 40%, they have some defects such as poor catalyst selectivity, low lifespan, difficulty in reactivation, and poor reproducibility of reaction results and so on.

SUMMARY

In order to solve above technology problems, the present application provides a method for preparing heptafluoroisobutyronitrile by a gas-phase catalysis, which has some benefits such as less water waste, less gas waste and less solid waste, a low cost, a high catalytic activity, a high product selectivity, a good reaction reproducibility, and a long catalyst life, and is suitable for an industrial continuous production.

The present application is achieved by following solutions.

The method for preparing heptafluoroisobutyronitrile by the gas-phase catalysis includes: removing one water molecule from heptafluoroisobutyramide gas to obtain heptafluoroisobutyronitrile by a catalyst. The catalyst includes an oxide of a siderophile element. The siderophile element includes a moderately siderophile element or a highly siderophile element. The moderately siderophile element is selected from the group consisting of tungsten, molybdenum, tin, gallium, and any combination thereof. The highly siderophile element is selected from the group consisting of osmium, iridium, ruthenium, rhenium, titanium and any combination thereof.

In the present application, since the moderately siderophile element includes characteristics such as a small ion radius, a high electrovalence, a strong polarization ability and so on, it is prone to forming complex anions which can form a relatively stable intermediate state with an enol form of an amide. Therefore, in some embodiments, the moderately siderophile element is selected from the group consisting of tungsten, molybdenum, tin, gallium, and any combination thereof. In some embodiments, the catalyst includes molybdenum oxide and/or tungsten oxide.

In the present application, a reaction temperature of a catalytic dehydration is in a range of 150 °C to 600 °C, a reaction pressure is in a range of 1 bar to 3 bars, a range of a residence time is greater than or equal to ($\tau$-1) s and less than or equal to ($\tau$+1) s. $\tau$ is obtained by following formula: $$\tau = \frac{\pi \times r^2 \times h \times k \times M \times P}{v \times \rho \times R \times T}$$ , in the formula, $r$ represents a radius of a reaction tube, $h$ represents an effective height of the reaction tube, i.e., a packing height of the catalyst, $k$ represents a packing coefficient of the catalyst, $M$ is 213.05 g/mol, $P$ represents a reaction pressure, $V$ represents an injection speed, $\rho$ is 1.517 g/mL, $R$ is 8.314 J/(mol·K), and $T$ represents a reaction temperature.

In some embodiments, a feed rate is in a range of 0.1 mL/min to 100 mL/min. A radius of a reacting tube is in a range of 10 mm to 300 mm. An effectively height of a reacting tube is in a range of 500 mm to 1000 mm. The residence time is in a range of 0.1 s to 50 s.

In some embodiments, the reaction temperature is in a range of 300 °C to 500 °C. The reaction pressure is in a range of 1 bar to 2 bars. A range of the residence time is greater than or equal to ($\tau$-0.1) s and less than or equal to ($\tau$+0.1) s.

In the present application, the catalyst is obtained by following steps: adding an ammonia solution with a high concentration of ammonia to a metal salt solution for precipitation, controlling a temperature to be less than or equal to 80 °C, standing and aging in a range of 12 h to 24 h; then evaporating, crystallizing, centrifuging, drying to obtain a precursor; and finally roasting to obtain the catalyst. The metal salt solution is obtained by dissolving or dispersing a raw material including the siderophile element in water (such as deionized water).

The raw material including the siderophile element is selected from the group consisting of metal acids, metal acid salts, metal alkoxides, chlorides, ammonium acid salts, and any combination thereof. In some embodiments, metal acids may be tungstic acids, molybdic acids, rhenium acids and so on. Metal acid salts may be sodium tungstate, sodium molybdate, sodium perrhenate, ruthenium acetate and so on. Metal alkoxides may be tetrabutyl titanate, tetraethyl titanate and so on. Chloride may be tungsten chloride, molybdenum chloride, tin chloride, titanium tetrachloride, osmium chloride, iridium chloride, ruthenium chloride and so on. Ammonium salts may be ammonium tungstate, ammonium paratungstate, ammonium molybdate, ammonium chloroosmate, ammonium chloroiridate and so on.

The precursor obtained by above methods is selected from the group consisting of hydroxides of the siderophile element, ammonium salts of the siderophile element, and any combination thereof.

In an embodiment, the catalyst in the present application may be obtained by following steps: dissolving and dispersing the raw material including the siderophile element, adding an ammonia solution with a high concentration of ammonia (wherein the concentration of the ammonia is in a range of 25% to 28%) for precipitation, controlling a temperature to be less than 80 °C, standing and aging in a range of 12 h to 24 h, evaporating and crystallizing for a range of 6 h to 12 h at a relative low temperature (less than 80 °C), centrifuging, drying in an oven at a range of 60 °C to 80 °C for a range of 12 h to 24 h to obtain a precursor; and roasting in a range of 300 °C to 500 °C for a range of 5 h to 24 h, grinding, forming, crushing, and screening to obtain the catalyst.

In an embodiment, a precursor including at least one of hydroxides of siderophores or ammonium salts of siderophores is prepared and obtained in the present application, including following steps: adding a mixture of an ammonia solution with a high concentration of ammonia and a second salt solution to a first salt solution for precipitation, controlling a temperature to be less than or equal to 80 °C, standing and aging in a range of 12 h to 24 h; then evaporating, crystallizing, centrifuging, and drying to obtain the precursor; and finally roasting to obtain the catalyst. The first salt solution is obtained by dissolving or dispersing at least one of metal acids of the siderophile element, metal acid salts of the siderophile element, metal alkoxides or chlorides of the siderophile element in water. The second salt solution is obtained by dissolving or dispersing the ammonium acid salts of the siderophile element in water.

In some embodiments, the first salt solution is obtained by dissolving and dispersing a material selected from the group consisting of tungsten acids, sodium tungstate, tungsten chloride, molybdic acids, sodium molybdate, molybdenum chloride, and any combination thereof in water. The second salt solution is obtained by dissolving and dispersing a material selected from the group consisting of ammonium tungstate, ammonium paratungstate, ammonium molybdate, and any combination thereof in water.

In the above obtained precursor, a molar ratio of the hydroxides of the siderophile element to the ammonium salts of the siderophile element is in a range of 1: 0.5 to 1: 1. In some embodiments, a molar ratio of the hydroxides of the siderophile element to the ammonium salts of the siderophile element is in a range of 1: 0.7 to 1: 1.

The precursor of the catalyst includes the hydroxides of the siderophile element and the ammonium salts of the siderophile element, such that the catalyst has a higher activity and a longer life.

The above catalyst is applied in a reaction of dehydrating heptafluoroisobutyramide to prepare heptafluoroisobutyronitrile. A catalyst life of the catalyst is in a range of 500 h to 2000 h. A space-time yield of the catalyst is in a range of 0.5 t/ (m$^3$cat·h) to 1.5 t/ (m$^3$cat·h). When a reaction temperature is in a range of about 150 °C to about 350 °C, a catalyst life of the catalyst is in a range of about 1000 h to about 2000 h. When a reaction temperature is in a range of about 350 °C to about 600 °C, a catalyst life of the catalyst is in a range of about 500 h to about 1500 h. In some embodiments, a catalyst life of the catalyst is in a range of about 1000 h to about 2000 h, and a space-time yield of the catalyst is in a range of 1.0 t/ (m$^3$cat·h) to 1.5 t/ (m$^3$cat·h).

Furthermore, in the present application, once the catalyst is deactivated, it can be easily regenerated. The present application further provides a step of regenerating the catalyst by blowing air and/or oxygen at a temperature in a range of 500 °C to 800 °C.

Compared with a related technology, advantages of the present application are as follows.

In the present application, the catalyst is utilized for catalytic dehydration of heptafluoroisobutyramide, such that a cost is low, a water waste, a gas waste and a solid waste are less, it is environmentally friendly. Furthermore, the catalyst has a good catalytic activity, a great product selectivity, a great reaction reproducibility, a long catalyst life, and is easy to be activated and regenerated.

BRIEF DESCRIPTION OF THE DRAWINGS

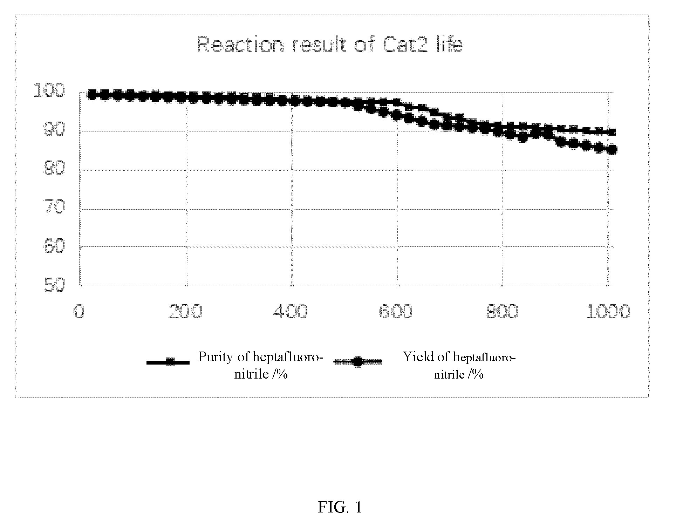

FIG. 1 is a diagram of a catalyst life of tungsten oxide (Cat2) in second embodiment of the present application.

DETAILED DESCRIPTION

The present application is further described in combination with a detailed explanation, and is not limited by the detailed explanation. Those skill in the art should realize that the present application covers all alternative, improved, and equivalent solutions that may be included within a scope of claims.

In the present application, examples are analyzed by a gas chromatography (GS). An analyzing apparatus is Shimadzu GC-2014. A chromatographic columnis GAS-Pro (60m X 0.32 mm X 1.0um). A GC analyzing method includes that a temperature of a detector is 250°C, a temperature of a vaporization chamber is 250 °C, a temperature of chromatographic column is kept at 60 °C for 5 min, increases to 85 °C by a speed of 10 °C/min, is kept at 85 °C for 3 min, increases to 250 °C by a speed of 10 °C/min, and is kept at 250 °C for 4 min; and a flow rate of a carrier gas (N$_2$) is at 1.05 mL/min, an air flow rate is at 300 mL/min, an hydrogen flow rate is at 30 mL/min, a split ratio is 50: 1, and an injection amount is 0.2 mL.

Preparation example 1

1.0 equivalent (eq) of molybdenum chloride was slowly and gradually added to 500 mL of deionized water, some precipitations were gradually strengthened, and a heat release temperature was controlled to be less than 80 °C, which was denoted as A solution. An ammonia solution with a high concentration of ammonia (9.0 eq and 28% volume concentration) was mixed with an ammonium molybdate (1.0 eq), which was denoted as B solution. The B solution was slowly added to the A solution stirred strongly, a heat release temperature was controlled to be less than 80 °C, which was stood and aged for 24 h, centrifuged, and dried in an oven at 80 °C for 12 h to obtain a precursor; and the precursor was a mixture of molybdenum hydroxide and ammonium molybdate. The precursor was calcined at 400 °C for 6 h, ground, formed, crushed, and sieved to obtain a molybdenum oxide by a protection of N$_2$, which was denoted as Cat1.

Preparation example 2

1.0 equivalent (eq) of tungsten chloride was slowly and gradually added to 500 mL of deionized water, some precipitations were gradually strengthened, and a heat release temperature was controlled to be less than 80 °C, which was denoted as an A solution. An ammonia solution with a high concentration of ammonia (10.0 eq and 28% volume concentration) was mixed with an ammonium paratungstate, which was denoted as a B solution. The B solution was slowly added to the A solution stirred strongly, a heat release temperature was controlled to be less than 80°C, which was stood and aged for 24 h, crystallized for 12 h at a relative low temperature (less than 50°C), centrifuged, and dried in an oven at 80 °C for 12 h to obtain a precursor, and the precursor was a mixture of molybdenum hydroxide and ammonium molybdate.

The precursor was calcined at 400 °C for 8 h, ground, formed, crushed, and sieved to obtain a molybdenum oxide by a protection of $N_2$, which was denoted as Cat2.

Preparation example 3

1.0 equivalent (eq) of tin chloride hydrate was slowly and gradually added to 500 mL of deionized water, some precipitations were gradually strengthened, and a heat release temperature was controlled to be less than 80 °C, an ammonia solution with a high concentration of ammonia (2.5 eq and 28% volume concentration) was slowly added to a reaction solution, a heat release temperature was controlled to be less than 80 °C, which was stood and aged for 24 h, centrifuged, and dried in an oven at 80 °C for 12 h to obtain a precursor, and the precursor was tin hydroxide. The precursor was calcined at 400 °C for 8 h, ground, formed, crushed, and sieved to obtain a tin oxide by a protection of $N_2$, which was denoted as Cat3.

Preparation example 4

1.0 equivalent (eq) of gallium chloride was slowly and gradually added to 500 mL of deionized water, some precipitations were gradually strengthened, and a heat release temperature was controlled to be less than 80 °C, an ammonia solution wih a high concentration of ammonia (3.5 eq and 28% volume concentration) was slowly added to a reaction solution, a heat release temperature was controlled to be less than 80 °C, which was stood and aged for 24 h, centrifuged, and dried in an oven at 80 °C for 12 h to obtain a precursor, and the precursor was gallium hydroxide. The precursor was calcined at 400 °C for 8 h, ground, formed, crushed, and sieved to obtain a tin oxide by a protection of $N_2$, which was denoted as Cat4.

Preparation example 5

1.0 equivalent (eq) of osmium chloride was slowly and gradually added to 500 mL of deionized water, some precipitations were gradually strengthened, and a heat release temperature was controlled to be less than 80 °C, an ammonia solution with a high concentration of ammonia (3.5 eq and 28% volume concentration) was slowly added to a reaction solution, a heat release temperature was controlled to be less than 80 °C, which was stood and aged for 24 h, centrifuged, and dried in an oven at 80 °C for 12 h to obtain a precursor, and the precursor was osmium hydroxide. The precursor was calcined at 400 °C for 8 h, ground, formed, crushed, and sieved to obtain a tin oxide by a protection of $N_2$, which was denoted as Cat5.

Preparation example 6

1.0 equivalent (eq) of iridium chloride was slowly and gradually added to 500 mL of deionized water, some precipitations were gradually strengthened, and a heat release temperature was controlled to be less than 80 °C, an ammonia solution with a high concentration of ammonia (4.5 eq and 28% volume concentration) was slowly added to a reaction solution, a heat release temperature was controlled to be less than 80 °C, which was stood and aged for 24 h, centrifuged, and dried in an oven at 80 °C for 12 h to obtain a precursor, and the precursor was iridium hydroxide. The precursor was calcined at 400 °C for 8 h, ground, formed, crushed, and sieved to obtain a tin oxide by a protection of $N_2$, which was denoted as Cat6.

Preparation example 7

1.0 equivalent (eq) of ruthenium chloride was slowly and gradually added to 500 mL of deionized water, some precipitations were gradually strengthened, and a heat release temperature was controlled to be less than 80 °C, an ammonia solution with a high concentration of ammonia (3.5 eq and 28% volume concentration) was slowly added to a reaction solution, a heat release temperature was controlled to be less than 80 °C, which was stood and aged for 24 h, centrifuged, and dried in an oven at 80 °C for 12 h to obtain a precursor, and the precursor was ruthenium hydroxide. The precursor was calcined at 400 °C for 8 h, ground, formed, crushed, and sieved to obtain a tin oxide by a protection of $N_2$, which was denoted as Cat7.

Preparation example 8

1.0 equivalent (eq) of rhenium chloride was slowly and gradually added to 500 mL of deionized water, some precipitatiosn was gradually strengthened, and a heat release temperature was controlled to be less than 80 °C, an ammonia solution with a high concentration of ammonia (5.5 eq and 28% volume concentration) was slowly added to a reaction solution, a heat release temperature was controlled to be less than 80°C, which was stood and aged for 24 h, centrifuged, and dried in an oven at 80 °C for 12 h to obtain a precursor, and the precursor was rhenium hydroxide. The precursor was calcined at 400 °C for 8 h, ground, formed, crushed, and sieved to obtain a tin oxide by a protection of $N_2$, which was denoted as Cat8.

Preparation example 9

1.0 equivalent (eq) of titanium tetrachloride was slowly and gradually added to 500 mL of deionized water, some precipitations were gradually strengthened, and a heat release temperature was controlled to be less than 80°C, an ammonia solution with a high concentration of ammonia (5.0 eq and 28% volume concentration) was slowly added to a reaction solution, a heat release temperature was controlled to be less than 80 °C, which was stood and aged for 24 h, centrifuged, and dried in an oven at 80 °C for 12 h to obtain a precursor, and the precursor was titanium hydroxide. The precursor was calcined for 8 h at 400 °C, ground, formed, crushed, and sieved to obtain a tin oxide by a protection of $N_2$, which was denoted as Cat9.

Preparation example 10

The present preparation example was substantially the same as preparation example 1, excepting that only the ammonia solution with a high concentration of ammonia was used for precipitation and was not mixed with the acid ammonium molybdate, a precursor was molybdenum hydroxide, and the precursor was calcined, ground, formed, crushed, and sieved to obtain a molybdenum, which was denoted as Cat10.

Comparative preparation example 1

A method for preparing a catalyst in the patent of CN109320436A was repeated, cobaltous oxide was taken as an example, the method included: dissolving cobalt nitrate hydrate in water, dropping an ammonia solution with a high concentration of ammonia for precipitation, adjusting pH to 7.5, aging 12 h, washing, filtering, drying in an oven at 80 °C for 36 h, roasting at 450°C for 8 h by a protection of $N_2$, obtaining cobaltous oxide, regarding cobaltous oxide as CatD1.

Comparative preparation example 2

1.0 equivalent (eq) of aluminum trichloride was slowly and gradually added to 500 mL of deionized water, some precipitation was gradually strengthened, and a heat release temperature was controlled to be less than 80 °C, an ammonia solution with a high concentration of ammonia (3.2 eq and 28% volume concentration) was slowly added to a reaction solution, a heat release temperature was controlled to be less than 80 °C, which was stood and aged for 24 h, centrifuged, and dried in an oven at 80 °C for 12 h to obtain a precursor, and the precursor was aluminum hydroxide. The precursor was calcined at 400 °C for 8 h, ground, formed, crushed, and sieved to obtain a tin oxide by a protection of $N_2$, which was denoted as CatD2.

Example 1

20 mL of molybdenum oxide (Cat1) obtained by the preparation example 1 was packed to a tubular reactor made of an inconel alloy with an inner diameter of 10 centimeters and an internal volume of 50 milliliters. Preheating furnace was increased to 150 °C, and circular water at 60 °C was inserted into a jacket of a gas liquid separation tank.

A reaction condition was as below: setting a reaction temperature at 350 °C, a reaction pressure at 1 bar, and a feed rate of heptafluoroisobutyramide at 0.5 mL/min, and circulating and obtaining a residence time of 6.5 s.

A reaction process was as below: heating and melting heptafluoroisobutyramide, gasifying in the preheating furnace through a high-temperature feed pump, catalyzing and dehydrating gasified heptafluoroisobutyramide via the tubular reactor, separating a reactant from a reaction tube of the tubular reactor through a gas-liquid separating tank, discharging a liquid material in the gas-liquid separating tank and circulating to the preheating furnace through a bypass, processing a gas through a two-stage cryogenic cooling, in which a first stage is to separate water at 0 °C, a second stage is to separate a heptafluoroisobutyronitrile producet, injecting the heptafluoroisobutyronitrile product to a steel cylinder, and washing non condensable gas with alkali and discharging.

The heptafluoroisobutyronitrile product was analyzed by GC, a purity of the heptafluoroisobutyronitrile product was 99.2%, and a yield of heptafluoroisobutyronitrile was 98.0%.

Example 2

This example was substantially the same as example 1, excepting that molybdenum oxide (Cat2) obtained by the preparation example 2 was used and packed, a circulated and obtained residence time was 6.5 s.

The heptafluoroisobutyronitrile product was analyzed by GC, a purity of the product is 99.5%, and a yield of heptafluoroisobutyronitrile is 99.1%

In a reaction condition of the present example, a catalyst life of molybdenum oxide (Cat2) was detected. FIG. 1 showed a reaction result diagram of molybdenum oxide (Cat2) continuously reacting for 1000 h, a method was a continuous reaction, sampling analysis and product weighing were conducted every 24 h. Referring to FIG. 1, after continuously reacting for 1000 h, a purity of the heptafluoroisobutyronitrile product was reduced from 99.5% to 89.5%, and a yield of heptafluoroisobutyronitrile was reduced from 99.1% to 85.0%.

Examples 3 to 10

Those examples were substantially the same as example 1, excepting that Cat3 to Cat 10 obtained by the preparation examples 3 to 10 were used and packed, respectively. The heptafluoroisobutyronitrile products of the examples 3 to 10 were analyzed by GC, respectively, and a purity of the heptafluoroisobutyronitrile product and a yield of heptafluoroisobutyronitrile were shown in table 1.

Comparative examples 1 to 2

Those comparative examples were substantially the same as example 1, excepting that CatD1 to CatD2 obtained by the preparation examples 1 to 2 were packed, respectively. Heptafluoroisobutyronitrile products of the comparative examples 1 to 2 were analyzed by GC, respectively, and a purity of the heptafluoroisobutyronitrile product and a yield of heptafluoroisobutyronitrile were shown in table 1.

Table 1 catalyzing reacting results of the catalysts

|  | Example/ Comparative example | Catalyst | Purity of heptafluoro-nitrile /% | Yield of heptafluoro-nitrile /% |
|---|---|---|---|---|
| Moderately siderophi -le element | Example 1 | Cat1 | 99.2 | 98.0 |
| | Example 2 | Cat2 | 99.5 | 99.1 |
| | Example 3 | Cat3 | 98.1 | 94.2 |
| | Example 4 | Cat4 | 81.0 | 97.5 |
| | Example 10 | Cat10 | 91.3 | 90.1 |
| Highly siderophi -le element | Example 5 | Cat5 | 90.8 | 84.5 |
| | Example 6 | Cat6 | 85.7 | 77.6 |
| | Example 7 | Cat7 | 88.4 | 73.8 |
| | Example 8 | Cat8 | 85.3 | 82.1 |
| | Example 9 | Cat9 | 81.0 | 97.5 |
| Other | Comparative example 1 | CatD1 | 6.9 | 36.3 |
| | Comparative example 2 | CatD2 | 64.2 | 8.7 |

Example 11

This example was substantially the same as example 1, excepting that a reaction temperature was set at 450°C, and a calculated and obtained residence time was 5.6 s.

A heptafluoroisobutyronitrile product was analyzed by GC, a purity of the heptafluoroisobutyronitrile product was 96.1%, and a yield of heptafluoroisobutyronitrile was 93.4%.

Example 12

This example was substantially the same as example 1, excepting that a feed rate of heptafluoroisobutyramide was at 1.0 mL/min, and a calculated and obtained residence time was 3.3 s.

A heptafluoroisobutyronitrile product was analyzed by GC, a purity of the heptafluoroisobutyronitrile product was 90.7%, and a yield of heptafluoroisobutyronitrile was 88.5%.

Example 13

This example was substantially the same as example 1, excepting that a reaction pressure was 2 bars, and a calculated and obtained residence time was 13.0 s.

A heptafluoroisobutyronitrile product was analyzed by GC, a purity of a heptafluoroisobutyronitrile product was 97.9%, and a yield of heptafluoroisobutyronitrile was 95.1%.

Example 14

A reaction condition of the present example used a reaction condition of example 16 of the patent of CN 109320436A, i.e., 20 mL tungsten oxide Cat2 obtained by the preparation example 2 was packed to a tubular reactor made of an inconel alloy with an inner diameter of 10 centimeters and an internal volume of 50 milliliters.

A reaction condition included: setting a reaction temperature at 400 °C, a reaction pressure at 0.1 MPa, and a time of heptafluoroisobutyramide contacting with the Cat2 to 10 s.

A reaction process included: condensing a reactant from a reaction tube through a bottle made of polytetrafluoroethylene, keeping a solid in a bottom of the bottle, removing water through a drying tube, flowing the reactant to a 200 mL steel cylinder made of 316 steel stainless, collecting a gas-phase production in a reaction system, i.e., heptafluoroisobutyronitrile.

After 24 h of continuous reaction, samples were taken every 2 hours, heptafluoroisobutyronitrile was analyzed by GC, a reaction result was shown in table 2.

Table 2: a reaction selectivity result diagram of catalyzing tungsten oxide for 24 h

| Time /h | Hexafluoropropylene /% | Heptafluoropropane /% | Heptafluoro-nitrile /% |
|---|---|---|---|
| 2 | 0.1 | 0.4 | 99.5 |
| 4 | 0.1 | 0.6 | 99.3 |
| 6 | 0.1 | 0.5 | 99.4 |
| 8 | 0.1 | 0.7 | 99.2 |
| 10 | 0.1 | 0.6 | 99.3 |
| 12 | 0.2 | 0.7 | 99.1 |
| 14 | 0.2 | 0.7 | 99.1 |
| 16 | 0.2 | 0.6 | 99.2 |
| 18 | 0.2 | 0.8 | 99.0 |
| 20 | 0.2 | 0.8 | 99.0 |
| 22 | 0.2 | 0.7 | 99.1 |
| 24 | 0.2 | 0.8 | 99.0 |

Comparative example 3

This comparative example was substantially the same as example 14, excepting that Cat2 was replaced by CatD1. After 24 h of continuous reaction, samples were taken every 2 hours, a product of the samples was analyzed by GC, and a reaction result was shown in table 3.

Table 3: a reaction selectivity result diagram of catalyzing tungsten oxide for 24 h

| Time /h | Hexafluoropropylene /% | Heptafluoropropane /% | Heptafluoro-nitrile/% |
|---|---|---|---|
| 2 | 22.6 | 13.4 | 62.9 |
| 4 | 24.5 | 17.2 | 56.7 |
| 6 | 20.8 | 18.6 | 59.1 |
| 8 | 26.4 | 15.6 | 56.2 |
| 10 | 27.8 | 15.6 | 55.9 |
| 12 | 28.8 | 17.6 | 52.3 |
| 14 | 28.9 | 16.3 | 53.4 |
| 16 | 27.8 | 19.6 | 51.4 |

(continued)

| Time /h | Hexafluoropropylene /% | Heptafluoropropane /% | Heptafluoro-nitrile/% |
|---------|------------------------|------------------------|------------------------|
| 18 | 28.5 | 20.1 | 50.3 |
| 20 | 29.2 | 23.6 | 45.2 |
| 22 | 29.9 | 24.1 | 43.1 |
| 24 | 28.6 | 27.6 | 42.1 |

Referring to FIG. 3, after 24 h of continuous reaction, the reaction selectivity continuously decreased. After reacting 24 h, the catalyst was severely coked, there were high temperature (400 °C) decomposition products of hexafluoropropene and heptafluoropropane.

Example 15

The catalyst reacted for 1000 h in example 2 was regenerated. A method further included a step of regenerating the catalyst including: blowing air to a reactor, and increasing a temperature to 500°C to burn off carbon deposits and maintain a hexagonal crystal morphology of the catalyst. A regenerated catalyst was detected by a reaction condition of example 2. The purity of a heptafluoroisobutyronitrile product was 98.1%, and a yield of heptafluoroisobutyronitrile was 92.7%.

**Claims**

1. A method for preparing heptafluoroisobutyronitrile by a gas-phase catalysis, comprising:
   removing one water molecule from heptafluoroisobutyramide gas to obtain heptafluoroisobutyronitrile by a catalyst; wherein the catalyst comprises an oxide of a siderophile element, the siderophile element comprises a moderately siderophile element or a highly siderophile element, wherein the moderately siderophile element is selected from the group consisting of tungsten, molybdenum, tin, gallium, and any combination thereof, and the highly siderophile element is selected from the group consisting of osmium, iridium, ruthenium, rhenium, titanium, and any combination thereof.

2. The method of claim 1, wherein the catalyst comprises the oxide of the siderophile element selected from the group consisting of tungsten, molybdenum, tin, gallium, and any combination thereof.

3. The method of claim 2, wherein the catalyst comprises molybdenum oxide and/or tungsten oxide.

4. The method of any one of claims 1 to 3, wherein a reaction temperature is in a range of 150 °C to 600 °C, a reaction pressure is in a range of 1 bar to 3 bar, a rang of a residence time is greater than or equal to ($\tau$-1) s and less than or equal to ($\tau$+1) s, wherein $\tau$ is obtained by following formula: $\tau = \dfrac{\pi \times r^2 \times h \times k \times M \times P}{v \times \rho \times R \times T}$, in the formula, $r$ represents a radius of a reaction tube, $h$ represents an effective height of the reaction tube, i.e., a packing height of the catalyst, $k$ represents a packing coefficient of the catalyst, $M$ is 213.05 g/mol, $P$ represents a reaction pressure, $V$ represents an injection speed, $\rho$ is 1.517 g/mL, $R$ is 8.314 J/(mol·K), and $T$ represents a reaction temperature.

5. The method of claim 4, wherein the reaction temperature is in a range of 300 °C to 500 °C, the reaction pressure is in a range of 1 bar to 2 bars, and a range of the residence time is greater than or equal to ($\tau$-0.1) s and less than or equal to ($\tau$+0.1) s.

6. The method of any one of claims 1 to 3, wherein the catalyst is obtained by following steps:

   adding an ammonia solution with a high concentration to a metal salt solution for precipitation, controlling a temperature to be less than 80 °C, standing and aging in a range of 12 h to 24 h, evaporating, crystallizing, centrifuging, drying to obtain a precursor, and roasting to obtain the catalyst;
   wherein the metal salt solution is obtained by dissolving or dispersing a raw material comprising the siderophile element in water.

7. The method of claim 6, wherein the raw material comprising the siderophile element is selected from the group consisting of metal acids, metal acid salts, metal alkoxides, chlorides, ammonium acid salts, and any combination thereof.

8. The method of claim 7, wherein the precursor is selected from the group consisting of hydroxides of the siderophile element, ammonium salts of the siderophile element, and any combination thereof.

9. The method of claim 6, wherein the catalyst is obtained by following steps:

adding a mixture of a stronger ammonia water and a second salt solution to a first salt solution for precipitation, controlling a temperature to be less than 80 °C, standing and aging in a range of 12 h to 24 h; then evaporating, crystallizing, centrifuging, drying to obtain the precursor; and finally roasting to obtain the catalyst; wherein the first salt solution is obtained by dissolving or dispersing at least one of metal acids of the siderophile element, metal acid salts of the siderophile element, metal alkoxides of the siderophile element or chlorides of the siderophile element in water; and the second salt solution is obtained by dissolving or dispersing the ammonium acid salts of the siderophile element in water.

10. The method of claim 9, wherein the first salt solution is obtained by dissolving and dispersing material selected from the group consisting of tungsten acids, sodium tungstate, tungsten chloride, molybdic acids, sodium molybdate, molybdenum chloride, and any combination thereof in water; and the second salt solution is obtained by dissolving and dispersing a material selected from the group consisting of ammonium tungstate, ammonium paratungstate, ammonium molybdate and any combination thereof in water.

11. The method of claim 9 or claim 10, wherein the precursor is a mixture selected from the group consisting of hydroxides of the siderophile element, ammonium salts of the siderophile element, and any combination thereof.

12. The method of claim 11, wherein in the precursor, a molar ratio of the hydroxides of the siderophile element to the ammonium salts of the siderophile element is in a range of 1: 0.5 to 1: 1.

13. The method of any one of claims 6 to 11, wherein a catalyst life of the catalyst is in a range of 500 h to 2000 h, and a space-time yield of the catalyst is in a range of 0.5 t/ ($m^3$cat·h) to 1.5 t/ ($m^3$cat·h).

14. The method of claim 13, further comprising a step of regenerating the catalyst by blowing air and/or oxygen at a temperature in a range of 500 °C to 800 °C.

FIG. 1

## INTERNATIONAL SEARCH REPORT

International application No.

**PCT/CN2023/132195**

| A. | CLASSIFICATION OF SUBJECT MATTER |
|---|---|

C07C253/20(2006.01)i; C07C255/10(2006.01)i; B01J21/06(2006.01)i; B01J23/08(2006.01)i; B01J23/14(2006.01)i; B01J23/28(2006.01)i; B01J23/30(2006.01)i; B01J23/36(2006.01)i; B01J23/46(2006.01)i

According to International Patent Classification (IPC) or to both national classification and IPC

| B. | FIELDS SEARCHED |
|---|---|

Minimum documentation searched (classification system followed by classification symbols)

C07C, B01J

Documentation searched other than minimum documentation to the extent that such documents are included in the fields searched

Electronic data base consulted during the international search (name of data base and, where practicable, search terms used)

CNTXT, ENTXTC, ENTXT, CNKI, STN(CAPLUS, CASREACT): 浙江省化工研究院有限公司, 中化蓝天集团有限公司, 毛崇智, 倪航, 蒋强, 杨汪松, 陈伟, 李伟, 七氟异丁酰胺 or 全氟异丁酰胺, 七氟异丁腈 or 全氟异丁腈, 钨 or 钼 or 锡 or 镓 or 铼 or 铱 or 钌 or 铼 or 钛, heptafluoroisobutyronitrile, heptafluoroisobutyramide, perfluoroisobutyronitrile, perfluoroisobutyramide, 42532-60-5, 662-20-4

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 109320436 A (BEIJING YUJI SCIENCE & TECHNOLOGY CO., LTD.) 12 February 2019 (2019-02-12)<br>entire document | 1-14 |
| A | CN 114057605 A (GUANGDONG LABORATORY OF CHEMISTRY AND FINE CHEMICAL INDUSTRY et al.) 18 February 2022 (2022-02-18)<br>entire document | 1-14 |
| A | CN 107935884 A (BEIJING YUJI SCIENCE & TECHNOLOGY CO., LTD.) 20 April 2018 (2018-04-20)<br>entire document | 1-14 |
| A | US 2015083979 A1 (3M INNOVATIVE PROPERTIES COMPANY) 26 March 2015 (2015-03-26)<br>embodiment 1 | 1-14 |

☑ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **29 January 2024** | **02 February 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/132195** |

### C. DOCUMENTS CONSIDERED TO BE RELEVANT

| Category* | Citation of document, with indication, where appropriate, of the relevant passages | Relevant to claim No. |
| --- | --- | --- |
| A | CN 111848444 A (JIUDING FLUORIN CHEMICALS CO., LTD. ZHANGPING FUJIAN) 30 October 2020 (2020-10-30)<br>      claims 1-7 | 1-14 |
| A | CN 114105820 A (ZHEJIANG RESEARCH INSTITUTE OF CHEMICAL INDUSTRY CO., LTD. et al.) 01 March 2022 (2022-03-01)<br>      entire document | 1-14 |

Form PCT/ISA/210 (second sheet) (July 2022)

**INTERNATIONAL SEARCH REPORT**
**Information on patent family members**

International application No.

**PCT/CN2023/132195**

| Patent document cited in search report | | | Publication date (day/month/year) | Patent family member(s) | Publication date (day/month/year) |
|---|---|---|---|---|---|
| CN | 109320436 | A | 12 February 2019 | None | |
| CN | 114057605 | A | 18 February 2022 | None | |
| CN | 107935884 | A | 20 April 2018 | None | |
| US | 2015083979 | A1 | 26 March 2015 | None | |
| CN | 111848444 | A | 30 October 2020 | None | |
| CN | 114105820 | A | 01 March 2022 | None | |

Form PCT/ISA/210 (patent family annex) (July 2022)

## REFERENCES CITED IN THE DESCRIPTION

**Patent documents cited in the description**

- US 15997808 A
- CN 108395382 A
- CN 110642750 A
- CN 107935884 A
- CN 114105820 A
- CN 114057605 A
- CN 109320436 A